# EUROPEAN PATENT SPECIFICATION

(11) **EP 0 455 306 B1**
(45) Date of publication and mention of the grant of the patent: **03.01.1996**
(21) Application number: 91201046.9
(22) Date of filing: 02.05.1991
(51) Int. Cl.: C07C 2/10

(54) **Olefin ethylation process**
Verfahren zur Olenfinethylierung
Procédé pour l'éthylation d'oléfines

(30) Priority: 02.05.1990 US 517772; 26.11.1990 US 617738
(43) Date of publication of application: 06.11.1991
(73) Proprietor: SHELL INTERNATIONALE RESEARCH MAATSCHAPPIJ B.V., NL-2596 HR Den Haag (NL)
(72) Inventor: Slaugh, Lynn Henry, Cypress, Texas 77429 (US)

(56) References cited:
- EP-A- 0 143 334
- US-A- 3 175 020
- US-A- 3 651 161
- US-A- 4 544 790

## Description

This invention relates to a process for the ethylation of olefins having at least one allylic hydrogen atom and from 8-22 carbon atoms in the presence of a catalyst.

Various processes for the alkylation of olefins employing alkali metal catalysts alone or supported on suitable carriers, together with certain promoters have previously been reported. US-A 2,492,693 discloses the use of an alkali metal (sodium) catalyst and a polynuclear aromatic hydrocarbon promoter for the intermolecular condensation of different monoolefins. US-A 3,651,161 discloses a process for the alkylation of compounds with an active hydrogen, allylic or benzylic, with the use of a sodium/pyrene charge transfer complex or a potassium/biphenyl charge transfer complex.

Processes for the dimerization of olefins employing supported alkali metal based catalysts are found in US-A 3,175,020 which discloses catalysts having potassium metal on an alumina carrier; and in US-A 4,609,637, US-A 4,661,466, US-A 4,544,790 and US-A 4,595,787 which teach the use of elemental alkali metals deposited on potassium carbonate catalyst supports.

A process for ethylating olefins having at least one allylic hydrogen atom by contacting one or more of said olefins with ethylene in the presence of an alumina based catalyst which is obtained by heating a mixture of potassium carbonate and an alumina support above at least 950 °C and subsequently adding at least one elemental alkali metal, is known from US-A 4,609,637.

The preparation of the catalyst in this process is cumbersome, in that the support requires heating at a very high temperature and in that the handling of elemental alkali metal(s) requires rigorous safety precautions to prevent fire hazards. Moreover, supported elemental alkali metal catalysts can not easily be regenerated.

The object of the present invention is the provision of a catalyst by which the disadvantages of the known olefin ethylation catalysts are obviated.

As a result of extensive research and experimentation it has now surprisingly been found that the use of an elemental alkali metal in the preparation of catalysts is superfluous and that aluminas without any additional ingredients are active catalysts for olefin ethylation.

The present invention, therefore, provides a process for ethylating olefins having at least one allylic hydrogen atom and from 8-22 carbon atoms by contacting one or more of said olefins with ethylene in the presence of an alumina based catalyst which is substantially free of elemental alkali metal.

It has also been found that the catalytic activity of the alumina is the highest when the content of sodium compounds is low in relation to the surface area of the alumina, preferably below 3x10⁻⁵ g sodium/m². In addition, particularly active ethylation catalysts can be obtained by supplying the alumina support with an alkali metal compound and calcining the resulting mixture. Such alkali metal compounds can be chosen which provide an alkali metal oxide upon calcination.

Compared with the known catalysts for the ethylation of olefins, the catalyst of the process of the invention is prepared under much less severe conditions, handled more easily and, moreover, rigorous safety precautions are not required. In general, the catalysts of the process of the invention can easily be regenerated, if needed and desired.

The present invention relates to a process for olefin ethylation by contacting an olefin with ethylene in the presence of a catalyst. The olefins are ethylated to prepare more-branched olefins having higher molecular weights. As used herein, "ethylation" and "ethylating" refer to the addition of one or more ethyl moieties (CH₃CH₂-) to an organic substrate which in this specification is an olefin. In a preferred embodiment, straight-chain olefins can be converted to mono-branched olefins having an ethyl group pendant to the main chain, or if desired, multi-branched products can be obtained.

The olefins suitable for use in the present invention are olefins having at least one allylic hydrogen atom. The olefins may be linear or cyclic and they may be branched. Preferably, the olefins are detergent range olefins which are olefins having from 8 to 22 carbon atoms, preferably from 8 to 18 carbon atoms. These olefins can be alpha olefins or internal olefins and they may be linear or branched. Single cut olefins or mixtures of olefins may also be used. In a particularly preferred embodiment, the olefin contains from 12 to 18 carbon atoms.

Preferred for use as olefin reactant for the practical reason of availability are commercial olefin products in the C₈ to C₂₂ range manufactured by the cracking of paraffin wax or by the oligomerization of ethylene, using procedures well known in the art. The oligomerization products are substantially of a linear structure. While most of such olefin products are comprised largely of alpha-olefins, higher linear internal olefins are also commercially produced, for example, by the chlorination-dehydrochlorination of paraffins, by paraffin dehydrogenation, and by isomerization of alpha-olefins. Commercially available substantially linear olefin products in the C₈ to C₂₂ range, whether predominantly internal olefins or alpha-olefins typically contain 70 percent by weight or more, most often 80 percent by weight or more, linear mono-olefins in a specified carbon number range (e.g., C₁₀ to C₁₂, C₁₁ to C₁₅, C₁₂ to C₁₃, C₁₅ to C₁₈, etc.), the remainder of the product being olefin of other carbon number or carbon structure, diolefins, paraffins, aromatics, and other impurities resulting from the synthesis process.

The alumina employed in the process of the invention is suitably a porous alumina support and can be any of the variety of available aluminas or alumina hydrates, such as alumina gel, activated alumina, gamma alumina. Very suitable aluminas are found to be those having a sodium content per unit surface area of less than 3 x 10⁻⁵ g/m², preferably less than 2.5 x 10⁻⁵ g/m² and more preferably less than 2 x 10⁻⁵ g/m², and having surface areas ranging from 10 m²/g to 500 m²/g, preferably from 50 m²/g to 400 m²/g. In a preferred embodiment, the alumina support is gamma alumina. Aluminas which are commercially readily available and which are suitable for use in the invention may have a surface area in the range of from 55 m²/g to 370 m²/g and may contain up to 5000 ppm sodium, up to 6000 ppm SO₄, up to 2000 ppm iron oxide (Fe₂O₃) and/or up to 6000 ppm Cl, measured as 8 weight fraction, relative to the weight of the alumina support.

The particularly active catalysts referred to hereinbefore are prepared for example, by impregnating or otherwise providing an alumina support with an alkali metal compound decomposable upon calcination to the oxide wherein the metal compound is selected from the group consisting of potassium, rubidium, cesium and mixtures thereof and then calcining the resultant composition at a temperature ranging from 450 °C to 750 °C, preferably from 500 °C to 700 °C. Calcining below the desired lower temperature limits results in catalysts which are not active, and calcining above the desired upper temperature limits results in excessive sintering with a resultant degradation of catalyst properties. It is thought that the decomposable compound(s) during the calcination react(s) after intermediate oxide formation with the alumina to form a metal aluminate. Suitable impregnating alkali metal compounds that decompose during calcination include, for example, carbonates, bicarbonates, hydroxides, chelates, alkoxylates and salts of other weak acids or salts of strong acids that decompose upon calcination such as the nitrates.

The calcination can be carried out in any atmosphere: vacuum, reducing, inert or oxidizing, with reducing and inert atmospheres being preferred. When the decomposable compound has an organic anionic moiety such as carboxylate, alkoxylate, chelate, etc., it is preferred to carry out the calcination in a reducing atmosphere such as hydrogen or an inert atmosphere such as nitrogen. Calcination times are not critical and depend on calcining temperatures, higher temperatures requiring shorter times and vice versa. Typical times range from 0.1 to 50 hours. The time-temperature combination selected should be such that the metal oxide or decomposable compound reacts almost completely with the alumina. A good result can be obtained when the catalyst is calcined in air and thereafter activated by flowing nitrogen over the catalyst at elevated temperature, about 575 °C, for about sixteen hours.

The alkali metals used to form the catalyst of the invention are potassium, rubidium and cesium. Combinations of these metals can also be utilized. The preferred alkali metal is potassium. Preferred impregnating materials for the alumina are potassium carbonates, potassium carboxylates and potassium nitrate. Salts of strong acids that do not completely decompose such as, for instance, sulphates and halides are less satisfactory.

Any conventional methods known for adding the metal oxide or decomposable compound to the alumina can be employed. A preferred method is to soak the alumina support pellets in an aqueous solution of the decomposable metal compound(s), dry the impregnated alumina, and then calcine at temperatures in the range of from 450 °C to 750 °C, preferably from 500 °C to 700 °C. Dry mixing can also be used. Since the metal oxide or decomposable compound is primarily reacting with the surface alumina, both external and internal pore surface, the maximum amount of impregnating compound that can be effectively utilized will depend on the surface area. Ordinarily, the molar ratio of metal added to alumina will range from 2:1 to 1:50, preferably from 1:1 to 1:25. Preferably, the weight of the metal oxide or decomposable compound added will range from 0.1 percent by weight to 30 percent by weight, more preferably from 1 percent by weight to 25 percent by weight, and most preferably, from 5 percent by weight to 20 percent by weight measured as the metal. In a preferred embodiment, a given portion of alumina pellets is impregnated with just enough aqueous solution of decomposable metal compound to fill the pore volume of the alumina, then dried at temperatures of up to 125 °C, and then calcined at temperatures in the range of from 450 °C to 750 °C, preferably from 500 °C to 700 °C.

The process of the invention can be carried out using either batch or continuous types of operation. The catalysts of the invention are particularly well suited for continuous or fixed bed operation. Suitable equipment such as, for example, autoclaves, tubular reactors and the like can be employed. Materials such as steel, stainless steel, glass-lined reactors and the like can be employed.

The reaction temperatures and pressures can vary depending on the olefin feed employed and the products desired. Typically, a temperature in the range of from 150 °C to 400 °C and a pressure in the range of from 14.8 bar (200 psig) to 207.9 bar (3000 psig) is suitable. The temperature is preferably in the range of from 150 °C to 300 °C, and more preferably in the range of from 200 °C to 250 °C. The pressure is preferably in the range of from 35.5 bar (500 psig) to 138.9 bar (2000 psig) and more preferably in the rang of from 56.2 bar (800 psig) to 83.8 bar (1200 psig). As the reaction temperatures and pressures are lowered, the conversion is lowered. If, for example, a monoethylated olefin is desired, it will be necessary to limit the feed conversion by lowering the temperature and pressure as the initial monoethylated olefin can undergo further ethylation. Temperatures in the range of from 150 °C to 250 °C and pressures in the range of from 56.2 bar (800 psig) to 83.8 bar (1200 psig) are most preferred as they result in a minimal amount of by-products from side reactions such as dimerization and polymerization.

The ethylation reaction is usually carried out in a liquid phase and if desired, solvents or diluents for the reactants can be used. Suitable diluents include saturated aliphatic hydrocarbons, e.g., pentane, hexane, cyclohexane; and aromatic compounds such as benzene and toluene.

The contact time required for the ethylation reaction depends upon several factors such as temperature, pressure, the level of conversion desired and the like. The length of time during which the ethylene and the olefins are contacted with the catalyst are usually between one hour and twenty hours, although shorter and longer contact times can be utilized. Preferably, the contact times are in the range of from two hours to ten hours. When the reaction is carried out in continuous fashion, the reactant/catalyst contact time may be expressed in terms of the liquid hourly space velocity (volume of reactants per volume of catalyst per hour, LHSV). The LHSV is suitably in the range of from 0.1 to 5.0 preferably from 0.5 to 1.0.

The relative amounts of the olefin reactant and ethylene are such that an ethylation product is produced which has the desired degree of branching in the carbon chain. In general, the detergent range olefins are contacted with sufficient ethylene for a sufficient time to yield a mixture of ethylated olefins characterised as the addition product of an average of between 1.0 and 1.3 mols of ethylene per mole of olefin. Addition of greater amounts of the detergent range olefins results in a larger portion of more-branched products. An excess of olefin reactant results in the production of ethylated olefins having primarily one added ethyl branch in the molecule. Preferably, the olefin reactant is used in such an excess that of the olefin reactant molecules, no more than 50%, preferably no more than 20%, and preferably, no more than 15% react with two or more ethylene molecules to yield ethylated olefins having more than one added ethyl branch. Maintaining a relatively low conversion, e.g., 10-20%, of olefin reactant during ethylation also aids in preventing the formation of polyethylated products.

The more-branched, higher molecular weight olefin products prepared from detergent range olefins according to the present process are useful in a wide variety of applications such as, for example, making a broad range of surfactants, including nonionic, anionic, cationic and amphoteric surfactants. The olefin products prepared according to the invention are particularly useful for making surfactant materials which have superior cold water detergency and better handling characteristics than their linear counterparts.

The ranges and limitations provided in this specification and claims are those which are believed to particularly point out and distinctly claim the present invention. It is, however, understood that other ranges and limitations which perform substantially the same function in substantially the same way to obtain the same or substantially the same result are intended to be within the scope of this invention as defined by the specification and claims.

The invention is illustrated by the following examples.

### Examples 1-11

These examples illustrate the use of alumina catalysts in a series of ethylation reactions in which 1-dodecene was contacted and reacted with ethylene to produce more-branched higher molecular weight olefins.

In Example 1, 30 millilitres of 1-dodecene was charged to a one-litre autoclave, under nitrogen blanket, together with 6 grams of a commercially available grade A-201 alumina (14-30 mesh) catalyst. After thoroughly flushing with nitrogen and evacuating, the autoclave was charged with 56.2 bar (800 psig) ethylene. The autoclave contents were then heated under stirring to 250 °C. As the ethylation reaction commenced, ethylene was added to maintain a total pressure in the range of 35.5 - 56.2 bar (500 - 800 psig). After 5 hours, the reaction was terminated and the autoclave contents were allowed to cool to room temperature. A total of 31 grams of reactants and catalyst were removed and then filtered to separate catalyst. The properties of the catalyst and the results are presented in Table I.

For Examples 2 and 3, the ethylation of 1-dodecene was carried out according to the same procedures, and using the same quantities of catalyst and olefin reactant, as described for Example 1, with the exception that the temperatures were 200 °C and 151 °C, respectively. The results are presented in Table I.

For Examples 4-8, the ethylation of 1-dodecene was carried out according to the procedures, and using the same quantities of catalyst and olefin reactant, as described for Example 1, with the exception that grade A-201 aluminas having different sodium contents were utilized as catalysts. The properties of the catalysts and the results are presented in Table I.

For Examples 9, 10 and 11, the ethylation of 1-dodecene was carried out according to the same procedures, and using the same quantities of catalyst and olefin reactant, as described for Example 1, with the exception that commercially available SCS 250 and SCS 59 types alumina were used as the catalysts. The properties of the catalysts and the results are presented in Table I.

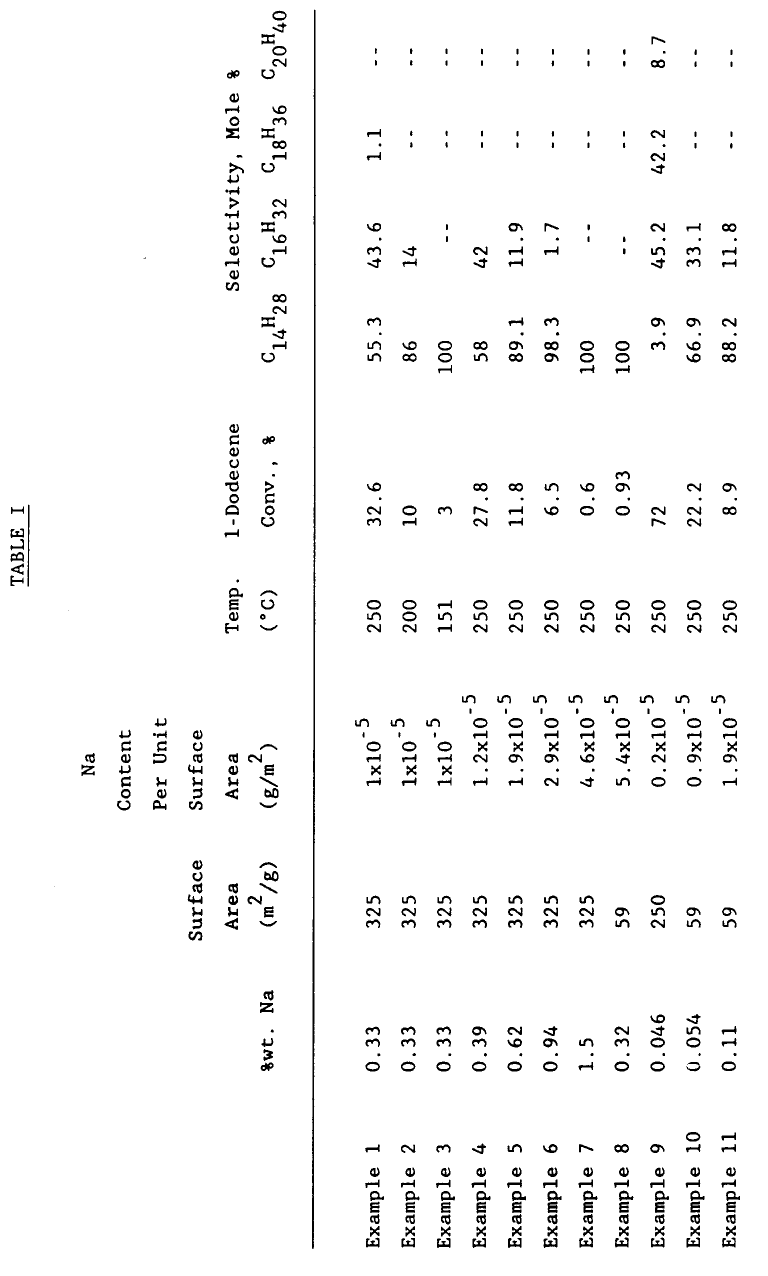

### Example 12

The following example illustrates the preparation of a particularly preferred catalyst for the process of the invention.

15 Grams of anhydrous K₂CO₃ were dissolved in 45 millilitres of deionized water. This solution was poured on 85 grams of a commercially available grade A-210 alumina (14-30 mesh) while stirring the latter. The volume of the solution and the weight of the alumina was proportioned to essentially fill the pores in the alumina without excess solution remaining after impregnation. The impregnated material was dried at 100 °C for 18 hours in air. The dried material was then calcined in a tube of flowing nitrogen for 16 hours at 575 °C. Analysis indicated that the composition contained about 9.3 percent by weight of potassium measured as the potassium metal.

Similar catalysts are prepared, for example by using solutions of potassium nitrate, potassium bicarbonate, potassium acetate and potassium nitrate.

### Examples 13-16

These examples illustrate the use of potassium carbonate/alumina catalysts in a series of ethylation reactions in which 1-dodecene was contacted and reacted with ethylene to produce more-branched higher molecular weight olefins.

In Example 13, 25 grams of 1-dodecene was charged to a one-liter autoclave, under nitrogen blanket, together with 6 grams of the potassium carbonate/alumina catalyst prepared as described above. After thoroughly flushing with nitrogen and evacuating, the autoclave was charged with 56.2 bar (800 psig) ethylene. The autoclave contents were then heated under stirring to 151 °C. As the ethylation reaction commenced, ethylene was added to maintain a total pressure in the range of from 35.5-56.2 bar (500-800 psig). After 5 hours, being equivalent to LHSV 1.0, the reaction was terminated by allowing the autoclave contents to cool to room temperature and releasing the pressure. A total of 31 grams of reactants and catalyst were removed and then filtered to separate the catalyst. The results are presented Table II.

For Examples 14, 15 and 16 the ethylation of 1-dodecene was carried out according to the same procedures, and using the same quantities of catalyst and olefin reactant, as described for Example 13, but using different reaction temperatures. The results and reaction temperatures are presented in Table II.

**TABLE II**

| | Temp. (°C) | 1-Dodecene Conv., % | Selectivity, Mole % | | |
|---|---|---|---|---|---|
| | | | C₁₄H₂₈ | C₁₆H₃₂ | C₁₈H₃₆ |
| Example 13 | 151 | 6.7 | 98.6 | 1.4 | 0 |
| Example 14 | 175 | 13.1 | 98.4 | 1.6 | 0 |
| Example 15 | 201 | 28.6 | 89.2 | 10.4 | 0.4 |
| Example 16 | 252 | 46.4 | 77.8 | 20.5 | 1.7 |

As can be seen in Tables I and II, relatively low conversions result in primarily mono-ethylated product. It can also be seen that deeper conversions result in an increase in branching.

In addition, the results in Table I show that the 1-dodecene conversions obtained in Examples 1-6 and 9-11 are much better than those obtained in Examples 7 and 8. In the latter Examples aluminas having sodium contents per unit surface area greater than about 3x10⁻⁵ g/m² were used.

## Claims

1. A process for ethylating olefin having at least one allylic hydrogen atom and from 8 to 22 carbon atoms by contacting one or more of said olefins with ethylene in the presence of an alumina based catalyst which is substantially free of elemental alkali metal.

2. A process as claimed in claim 1, characterized in that the alumina based catalyst has a sodium content per unit surface area of less than 3x10⁻⁵ g/m².

3. A process as claimed in claim 2, characterized in that the alumina based catalyst has a sodium content per unit surface area of less than 2x10⁻⁵ g/m².

4. A process as claimed in any of claims 1 - 3, characterized in that the alumina based catalyst has a surface area in the range of from 50 m²/g to 400 m²/g.

5. A process as claimed in any of claims 1 - 4, characterized in that the alumina based catalyst is obtainable by impregnating an alumina support with at least one metal compound which is decomposable to an oxide upon calcination wherein said metal is selected from the group consisting of potassium, rubidium, cesium and mixtures thereof, and subsequently calcining the impregnated support at a temperature of from 500 to 700 °C.

6. A process as claimed in claim 5, characterised in that the quantity of the metal compound(s) added to the alumina support ranges from 5 to 20 percent by weight measured as the metal.

7. A process as claimed in claim 5 or 6, characterised in that the metal compound(s) added to the alumina support is/are selected from the group consisting of potassium carbonates, potassium carboxylates, potassium nitrate and mixtures thereof.

8. A process as claimed in claim 7, characterised in that the metal compound added to the alumina support is potassium carbonate.

9. A process as claimed in any of claims 1-8, characterised in that the olefins comprise linear and/or branched olefins having from 8 to 18 carbon atoms.

10. A process as claimed in any of claims 1-9, characterised in that said ethylation is carried out at a temperature in the range of from 150 °C to 300 °C and a pressure in the range of from 35.5 bar (500 psig) to 138.9 bar (2000 psig).

## Patentansprüche

1. Verfahren zur Ethylierung von Olefinen mit mindestens einem allylischen Wasserstoffatom und mit 8 bis 22 Kohlenstoffatomen, bei dem man eines oder mehrere der Olefine in Gegenwart eines auf Aluminiumoxid basierenden Katalysators, der weitgehend frei von elementarem Alkalimetall ist, mit Ethylen kontaktiert.

2. Verfahren nach Anspruch 1, dadurch gekennzeichnet, daß der auf Aluminiumoxid basierende Katalysator je Oberflächeneinheit einen Natriumgehalt von weniger als 3x10⁻⁵ g/m² aufweist.

3. Verfahren nach Anspruch 2, dadurch gekennzeichnet, daß der auf Aluminiumoxid basierende Katalysator je Oberflächeneinheit einen Natriumgehalt von weniger als 2x10⁻⁵ g/m² aufweist.

4. Verfahren nach einem der Ansprüche 1 - 3, dadurch gekennzeichnet, daß der auf Aluminiumoxid basierende Katalysator eine Oberfläche im Bereich von 50 m²/g bis 400 m²/g aufweist.

5. Verfahren nach einem der Ansprüche 1 - 4, dadurch gekennzeichnet, daß man den auf Aluminiumoxid basierenden Katalysator durch Imprägnieren eines Aluminiumoxidträgers mit mindestens einer Metallverbindung, welche beim Kalzinieren zu einem Oxid abbaubar ist, wobei man das Metall aus der Gruppe bestehend aus Kalium, Rubidium, Caesium und deren Mischungen auswählt, und nachfolgendes Imprägnieren des kalzinierten Trägers bei einer Temperatur von 500 bis 700°C erhält.

6. Verfahren nach Anspruch 5, dadurch gekennzeichnet, daß die dem Aluminiumoxidträger zugesetzte Menge der Metallverbindung(en) im Bereich von 5 bis 20 Gewichtsprozent, bezogen auf das Metall, liegt.

7. Verfahren nach Anspruch 5 oder 6, dadurch gekennzeichnet, daß man die dem Aluminiumoxidträger zugesetzte(n) Metallverbindung(en) aus der Gruppe bestehend aus Kaliumcarbonaten, Kaliumcarboxylaten, Kaliumnitrat und deren Mischungen auswählt.

8. Verfahren nach Anspruch 7, dadurch gekennzeichnet, daß es sich bei der dem Aluminiumoxidträger zugesetzten Metallverbindung um Kaliumcarbonat handelt.

9. Verfahren nach einem der Ansprüche 1 - 8, dadurch gekennzeichnet, daß es sich bei den Olefinen um lineare und/oder verzweigte Olefine mit 8 bis 18 Kohlenstoffatomen handelt.

10. Verfahren nach einem der Ansprüche 1 - 9, dadurch gekennzeichnet, daß man die Ethylierung bei einer Temperatur im Bereich von 150°C bis 300°C und einem Druck im Bereich von 35,5 bar (500 lbf/in²) bis 138,9 bar (2000 lbf/in²) durchführt.

## Revendications

1. Procédé d'éthylation d'oléfines possédant au moins un atome d'hydrogène allylique et de 8 à 22 atomes de carbone par la mise en contact d'une ou plusieurs desdites oléfines avec de l'éthylène en présence d'un catalyseur à base d'alumine, sensiblement dépourvu de métal alcalin élémentaire.

2. Procédé suivant la revendication 1, caractérisé en ce que le catalyseur à base d'alumine possède une teneur en sodium par unité de surface spécifique inférieure à 3 x 10⁻⁵ g/m².

3. Procédé suivant la revendication 2, caractérisé en ce que le catalyseur à base d'alumine possède une teneur en sodium par unité de surface spécifique inférieure à 2 x 10⁻⁵ g/m².

4. Procédé suivant l'une quelconque des revendications 1 à 3, caractérisé en ce que le catalyseur à base d'alumine possède une surface spécifique qui varie de 50 m²/g à 400 m²/g.

5. Procédé suivant l'une quelconque des revendications 1 à 4, caractérisé en ce que le catalyseur à base d'alumine peut s'obtenir par l'imprégnation d'un support d'alumine par au moins un composé de métal qui est décomposable en un oxyde par calcination, où on choisit le métal en question dans le groupe formé par le potassium, le rubidium, le césium et leurs mélanges et la calcination subséquente du support imprégné à une température de 500 à 700°C.

6. Procédé suivant la revendication 5, caractérisé en ce que la quantité du ou des composés de métaux ajoutée au support d'alumine varie de 5 à 20% en poids, mesurés sous forme du métal.

7. Procédé suivant la revendication 5 ou 6, caractérisé en ce que le ou les composés de métaux ajoutés au support d'alumine sont choisis dans le groupe formé par les carbonates de potassium, les carboxylates de potassium, le nitrate de potassium et leurs mélanges.

8. Procédé suivant la revendication 7, caractérisé en ce que le composé de métal ajouté au support d'alumine est le carbonate de potassium.

9. Procédé suivant l'une quelconque des revendications 1 à 8, caractérisé en ce que les oléfines sont constituées d'oléfines linéaires et/ou ramifiées possédant de 8 à 18 atomes de carbone.

10. Procédé suivant l'une quelconque des revendications 1 à 9, caractérisé en ce que l'on entreprend ladite éthylation à une température qui fluctue de 150°C à 300°C et sous une pression qui varie de 35,5 bars (500 psi manométriques) à 138,9 bars (2000 psi manométriques).
